# EUROPÄISCHE PATENTANMELDUNG

(11) **EP 4 201 202 A1**
(43) Veröffentlichungstag der Anmeldung: **28.06.2023**
(21) Anmeldenummer: 21216725.8
(22) Anmeldetag: 22.12.2021
(51) Int. Cl.: A01K 67/033

(54) **ZUCHTVORRICHTUNG FÜR INSEKTENLARVEN**

(71) Anmelder: SmartBreed AG, 5621 Zufikon (CH)
(72) Erfinder: Bertschi, Patrik, 5621 Zufikon (CH); Bertschi, Christoph, 5621 Zufikon (CH); Bertschi, Adrian, 5621 Zufikon (CH)
(74) Vertreter: P&TS SA (AG, Ltd.)

(57) **Zusammenfassung**

Die Erfindung betrifft eine Zuchtvorrichtung (1) zur Anzucht von Insektenlarven mit einem Zuchtbehälter (2) aufweisend eine obere Eingabeöffnung (21) zum Befüllen des Zuchtbehälters mit Füllmaterial und eine in einem unteren Bereich (B1) des Zuchtbehälters befindlichen Ausgabeöffnung (22). Die Eingabeöffnung (21) und die Ausgabeöffnung sind (22) derart angeordnet, dass ein Füllmaterialfluss von der Eingabeöffnung zur Ausgabeöffnung erstellt wird. Der Zuchtbehälter weist eine Vielzahl von formstabilen und/oder formresistenten Druckschutzelementen (24, 25) auf, die dazu ausgebildet sind, einen Teil des Füllmaterials von dem Druck eines Teils des darüberliegenden Füllmaterials zu schützen.

## Beschreibung

### Technisches Gebiet

Die Erfindung bezieht sich auf eine Zuchtvorrichtung zur Anzucht von Insekten, und im speziellen von Insektenlarven.

### Stand der Technik

Insekten sind ein Bestandteil der natürlichen Nahrung vieler Nutztiere, wie beispielsweise Schweine, Hühner und Fische, und stellen eine wichtige Proteinquelle für diese Tiere dar. Das tierische Protein kann von Nutztieren sehr gut aufgenommen werden und unterstützt deren Wachstum. Da Insekten Lebensmittelreste und -nebenströme verwerten können und als Futtermittel den Nutztieren wieder verfügbar machen können, sind sie eine qualitativ hochwertige Proteinquelle. Insektenfutter stellt daher ein artgerechtes, proteinreiches und ausgewogenes Futtermittel für diese Tiere dar.

Insekten durchlaufen verschiedene Stadien in ihrem Lebenszyklus: adulte Tiere legen Eier, aus denen Insektenlarven schlüpfen, die heranwachsen und sich verpuppen. Aus den Puppen schlüpfen dann wiederum adulte Tiere. Herangewachsenen Insektenlarven sind als Tierfutter besonders attraktiv, da die Larven einen hohen Proteingehalt aufweisen.

Die Larven des Mehlkäfers, auch Mehlwürmer genannt, sowie Larven der schwarzen Soldatenfliege stellen beliebtes Futtermittel dar. Diese Larven bestehen aus 47 Gew % bis 52 Gew % tierischem Protein, aus 31 Gew % bis 44 Gew % Fett und aus 5 Gew % bis 15 Gew % Ballaststoffen. Geschlüpfte Mehlwürmer wachsen über etwa acht Wochen zu einer Grösse von etwa 2 cm heran. Die herangewachsenen Mehlwürmer werden vor ihrem Verpuppen geerntet und als Tierfutter verwertet.

Die Anzucht der Mehlwürmer erfolgt in einem Substrat, welches sie gleichzeitig auch fressen. Das Substrat ist vorzugsweise Weizenkleie, ein Mühlenachprodukt, bestehend aus den nicht verwertbaren Rückständen von Weizenschalen nach der Mehlproduktion. Mehlwürmer benötigen ausserdem Feuchtfutter, dem sie das benötigte Wasser entnehmen.

Weil Mehlwürmer während ihrer Anzucht viel Wärme produzieren und Sauerstoff für die Atmung benötigen, werden sie üblicherweise in einem Substrat mit einer Tiefe von maximal 10 cm gezüchtet. Bei tieferer Substrattiefe kann die Abwärme und das durch die Atmung produzierte Kohlendioxid nicht ausreichend entweichen und beeinträchtigt die Sauerstoffzufuhr an die Zuchttiere.

Erfahrungen aus der Mehlwurmzucht zeigen, dass sich die Insektenlarven nur in der obersten Schicht aufhalten. Für die Zucht bedeutet dies eine besondere Herausforderung, da Mehlwürmer in vielen kleinen Behältern mit einer Tiefe von max. 10cm gezüchtet werden müssen. Aus diesem Grund werden für die Mehlwurmzucht herkömmlicherweise flache Behälter verwendet.

Für die Ernte, oder um frisches Substrat und Feuchtfutter beizugeben, müssen die Behälter jeweils einzeln hervorgenommen werden, was mit einem enormen Arbeitsaufwand verbunden ist. Die Mehlwurm Anzucht im industriellen Massstab bedarf daher hohen Investitionskosten und Unterhaltskosten.

Der mit der Zucht verbundene hohe Arbeits- und Personalaufwand macht eine Nutzung von Mehlwürmern als lokales und nachhaltiges Tierfutter ökonomisch uninteressant. Automatisierte Ansätze für die Fütterung und Entleerung der Behälter sind zwar bekannt, allerdings basieren diese Lösungen auf kostenintensiver Robotertechnik und benötigen viel Platz.

Es besteht daher ein dringender Bedarf and Lösungen für die Anzucht von Insektenlarven, die diese Nachteile bestehender Zuchtmethoden beseitigen.

### Darstellung der Erfindung

Es ist ein Ziel der Erfindung, ein Zuchtsystem zur industriellen Anzucht von Insektenlarven zu finden, das platzsparend ist.

Der Arbeitsaufwand für die Zucht der Insektenlarven soll gegenüber konventionellen Zuchtmethoden verringert werden.

Das Zuchtsystem soll energiesparend im Vergleich zu automatisierten herkömmlichen Methoden sein.

Das Zuchtsystem soll des Weiteren kostengünstig sein, um eine lokale Anzucht von Insektenlarven, vorzugsweise in der Nähe von den Agrarnebenprodukten, Lebensmittelabfällen oder in einem Nutztierzuchtbetrieb zu ermöglichen.

Erfindungsgemäss werden eines oder mehrere dieser Ziele durch die unabhängigen Ansprüche erreicht. Weitere vorteilhafte Ausführungsformen sind in den Unteransprüchen angegeben.

Spezifisch werden die Ziele durch eine Zuchtvorrichtung für Insektenlarven erreicht, die einen Zuchtbehälter mit einer oberen Eingabeöffnung zum Befüllen mit Füllmaterial und mit in einem unteren Bereich des Zuchtbehälters befindlichen Ausgabeöffnung aufweist, erreicht. Das Füllmaterial umfasst vorzugsweise ein Substrat für die Insekten, beispielsweise Weizenkleie, Feuchtfutter, sowie Insektenzuchttiere.

Die Eingabeöffnung und die Ausgabeöffnung sind derart angeordnet, dass ein Füllmaterialfluss von der Eingabeöffnung zur Ausgabeöffnung erstellt wird. Der Füllmaterialfluss wird im Wesentlichen, vorzugsweise ausschliesslich, von dem Eigengewicht der Füllmasse bewirkt.

Um einen auf der Schwerkraft beruhenden Materialdurchfluss zu bewirken, muss die Eingabeöffnung oberhalb der Ausgabeöffnung angeordnet sein. Idealerweise ist der Bereich zwischen der Eingabe- und der Ausgabeöffnung im Wesentlichen perpendikulär zur Erdoberfläche angeordnet.

Um zu vermeiden, dass der Druck das Wachstum oder das Überleben von Larven in tiefergelegenen Bereichen des Füllmaterials beeinträchtigt, ist der Zuchtbehälter mit einer Vielzahl von formstabilen und/oder formresistenten Druckschutzelemente ausgestattet.

Diese Druckschutzelemente sind derart ausgebildet, den Druck eines Teils der Masse des Füllmaterials auf zumindest einen Teil der darunterliegende Masse des Füllmaterials zu reduzieren. Der besagte Teil der Masse des Füllmaterials liegt zumindest einem Abschnitt des Druckschutzelement, vorzugsweise einer Wand, auf. Das Druckschutzelement oder der Abschnitt des Druckschutzelementes, dem dieser Teil der Füllmasse aufliegt, stützt diesen Teil der Füllmasse. Der Pressdruck auf zumindest einen Teil der Füllmasse, der unterhalb des Druckschutzelements oder dessen besagten Abschnittes liegt, ist somit verringert.

Die Druckschutzelement dazu ausgebildet, Schutzzonen zu schaffen, in denen der durch die Masse des darüberliegenden Füllmaterials hervorgerufene Druck reduziert ist.

Diese Druckschutzelemente sind im Innenraum des Zuchtbehälters angeordnet. Druckschutzelemente können über den gesamten Innenraum des Zuchtbehälters verteilt sein.

Für die industrielle Produktion von Insektenlarven kann der hier beschriebene Zuchtbehälter eine Vielzahl von den oben erwähnten, relativ kleinen, herkömmlichen Behältern, in denen Larven in einer Substrattiefe von weniger als 10 cm gezüchtet werden, ersetzen. Da in diesem Fall das Volumen des im Zuchtbehälter vorgelegten Füllmaterials wesentlich vergrössert ist, kann sich ein signifikanter, durch die Füllmasse bedingter Druck aufbauen, der vor allem auf tieferliegendes Füllmaterial einwirkt.

Die Druckschutzelemente wirken diesem Druck entgegen, indem sie lokale Schutzzonen schaffen. Die Druckschutzelemente schützen einen Teil des Füllmaterials gegen den Druck eines Teils des darüberliegenden Füllmaterials.

Die lokale Reduktion des Pressdruckes der Masse des Füllmaterials mittels der Druckschutzelemente, trägt wesentlich zu verbesserten Anzuchtbedingungen für Insektenlarven bei. Auf Grund dieser verbesserten Anzuchtbedingungen kann der hier beschriebene Zuchtbehälters ein wesentlich grösseres Füllvolumen aufweisen als herkömmliche Zuchtbehälter.

Ein Zuchtbehälter der vorliegenden Erfindung kann beispielsweise ein Füllvolumen von 6 m³ bis 250 m³ aufweisen. Es ist somit möglich grosse Mengen an Insektenlarven in einem einzigen Zuchtbehälter zu produzieren. Die mit der Handhabung vieler kleinerer Behälter verbundene Arbeitsaufwand und Kosten können somit signifikant reduziert werden.

Der Zuchtbehälter kann beispielsweise eine zylindrische oder konische Form aufweisen. Der Zuchtbehälter kann ein Silo sein.

Vorzugsweise ist die Ausgabeöffnung mit einer Regulierungsvorrichtung, beispielsweise einer verstellbaren Verschlussklappe oder einer Transportschnecke, versehen. Mittels der Regulierungsvorrichtung kann die Ausgabe des Füllmaterials durch die Ausgabeöffnung kontrolliert, reguliert und/oder gestoppt werden.

Durch eine kontrollierte Materialausgabe aus der Ausgabeöffnung kann der Materialfluss des Füllmaterials reguliert werden. Wenn die Regulierungsvorrichtung, beispielsweise die Verschlusskappe, die Ausgabeöffnung verschliesst, ist der Materialfluss gestoppt. Wird die Regulierungsvorrichtung geöffnet, wird Material durch die Ausgabeöffnung abgegeben und das darüberliegende Füllmaterial bewegt sich in Richtung der Ausgabeöffnung.

Eine Ausgabe von Material kann beispielsweise zu bestimmten Zeitpunkten und/oder für eine bestimmte Dauer erfolgen. Dadurch wird jeweils ein Volumen der Füllmasse zu verschiedenen Zeiten geernteten, wodurch ein temporärer Materialfluss erstellt wird. In den Perioden zwischen den Ausgabezeiten stoppt der Materialfluss.

Der Materialfluss kann allerdings auch zumindest teilweise kontinuierlich erfolgen. Der Materialfluss kann beispielsweise durch eine verstellbare Durchlaufgeschwindigkeit des Materials durch die Ausgabeöffnung reguliert werden. Zu diesem Zweck kann beispielsweise eine Regulierungsvorrichtung zum Einstellen des Durchmessers der Ausgabeöffnung oder der Form der Ausgabeöffnung vorgesehen sein. Die Ausgabeöffnung kann beispielsweise nur teilweise und/oder zu einem bestimmten Ausmass von der Regulierungsvorrichtung freigegeben werden, sodass der Materialdurchfluss eingestellt werden kann.

Die Durchflussrate und der Durchflussmodus, das heisst temporär oder kontinuierlich, kann entsprechen der gezüchteten Insektenart gewählt werden. Die Anzucht von Larven mancher Insektenarten, wie beispielsweise die Larven der schwarzen Soldatenfliege, bedürfen längerer Perioden in statischem Füllmaterial.

Für andere Arten wiederum sind kürzere stagnierende Perioden, oder ein kontinuierlicher Füllmaterialfluss besser geeignet.

Die Druckschutzelemente können verschiedenste Formen annehmen.

Die Druckschutzelemente können beispielsweise Netze sein. Die Druckschutzelemente können auch zusätzliche Oberflächen im Inneren des Schutzbehälters sein. Diese Oberflächen können beispielsweise perforiert sein.

Die Druckschutzelemente sind derart im Inneren des Zuchtbehälters angeordnet, dass zumindest einen Teil des Gewichts eines Teils der Masse des Füllmaterials zu tragen, um so den Druck auf einen unterhalb liegenden Teil der Füllmasse zu reduzieren.

Netzförmige Druckschutzelemente bieten den zusätzlichen Vorteil, dass sie die Eiablage der adulten Insekten ermöglichen. Dadurch kann der Zuchtbehälter kontinuierlich bestückt werden, ohne dass eine externe Zugabe von Eiern oder Junglarven unbedingt erforderlich ist. Rauhe Oberflächen flächiger Druckschutzelemente sind ebenfalls als Strukturen für die Eiablage von adulten Insekten geeignet.

Druckschutzelemente sind vorzugsweise Strukturelemente mit einer Wand, die über einem Teil des Füllmaterials liegt, der nicht dem Gewicht der sich über der Wand befindlichen Füllmaterials ausgesetzt ist. Die Druckschutzelemente schützen somit einen Teil der Füllmasse vor dem Gewicht der oberhalb-gelegenen Masse des Füllmaterials.

In einer möglichen Ausführungsform sind die Druckschutzelemente dachförmige Umleitstrukturen, beispielsweise giebelförmige, kegeldachförmige oder gewölbte Elemente, deren Form ein Abgleiten des über dem Druckschutzelement befindlichen Füllmaterials entlang der Oberfläche des Druckschutzelements ermöglicht.

Die Schrägflächen dachförmiger Druckschutzelemente weisen vorzugsweise einen Neigungswinkel von 30° bis zu 70° in Bezug auf eine zur Erdanziehungskraft perpendikuläre Ebene auf.

Ein Vorteil dieser Ausführungsform ist, dass sich unterhalb der dachförmigen Struktur Lufttaschen bilden können. Larven, die eine gute Luftzufuhr benötigen, wie beispielsweise Mehlwürmer, finden unter diesen dachförmigen Strukturen gute Wachstumsbedingungen und können sich in Bereichen unter den dachförmigen Strukturen ansammeln.

Druckelemente in dieser Ausführung sind vorzugsweise im Zuchtbehälter angebracht. Die Druckschutzelemente können beispielsweise an eine im Innenraum befindliche Trägerstruktur, beispielsweise an einem Gestänge oder an einer zentralen Säule, angebracht sein.

Die Druckschutzelemente können auch direkt oder indirekt an der Innenwand des Zuchtschutzbehälters befestigt sein.

Befestigte Druckschutzelemente können statisch sein. Befestigte Druckschutzelemente können aber auch beweglich sein.

Druckschutzelemente können beispielsweise an einer rotierenden Trägerstruktur, beispielsweise einer zentralen Säule, befestigt sein. Die Druckschutzelemente rotieren somit gemeinsam mit der Trägerstruktur, wodurch das Füllmaterial aufgelockert wird. Dies trägt zu einer verbesserten Durchlüftung des Füllmaterials bei.

Des Weiteren kann es sich für Durchlüftung und/oder Materialdurchfluss als vorteilhaft erweisen, vibrierende Druckschutzelemente vorzusehen. Eine Vibration der Druckschutzelemente bewirkt eine zusätzliche Auflockerung des Füllmaterials und wirkt dem Druckaufbau entgegen. Eine Vibration der Druckschutzelemente verbessert auch die Belüftung des Füllmaterials.

In einer weiteren möglichen Ausführungsform liegen die Druckschutzelemente als mit Füllmaterial befüllbare Druckschutzgehäuse vor. Diese Druckschutzgehäuse sollten in den Zuchtbehälter eingefüllt, und die durch die Ausgabeöffnung des Zuchtbehälters entlassen werden können. Die Druckschutzgehäuse sind dynamische Elemente, die in Bezug zueinander und in Bezug auf den Zuchtbehälter beweglich sind.

Vorzugsweise weisen die Druckschutzgehäuse eine Vielzahl von Öffnungen auf, die ein Eindringen und Auslaufen von Füllmaterial ermöglichen.

Druckschutzgehäuse können zum Beispiel hohle Bälle sein. Allerdings können Druckschutzgehäuse auch als andere hohle Formen, wie beispielsweise Würfel, ausgeführt sein. Die Erfindung ist nicht auf eine spezifische Form der Druckschutzgehäuse oder auf eine Form der mehrfachen Öffnungen limitiert.

Druckschutzgehäuse eignen sich besonders für die Anzucht von Insektenlarven, die nasses Substrat benötigen. Lufttaschen sind für diese Arten nicht unbedingt notwendig, sodass die Druckschutzgehäuse teilweise oder vollständig mit nassem Substrat und Insektenzuchttieren angefüllt sein können. Befüllte Druckschutzgehäuse können, optional mit zusätzlichem Füllmaterial, in den Zuchtbehälter eingefüllt werden.

Druckschutzgehäuse können zur Ernte durch die Ausgabeöffnung ausgegeben werden. Nach der Ernte können die Larven und das verbleibende Substrat, sowie Abfall aus den Druckschutzgehäusen entfernt werden. Neu bestückte Druckschutzgehäuse können wieder zur weiteren Anzucht mit frischen Füllmaterial ausgestattet werden und in den Zuchtbehälter eingeführt werden.

Optional können die Druckschutzgehäuse vor dem Bestücken gewaschen werden. Ein Waschritt ist aber nicht unbedingt erforderlich. In den ungewaschenen Druckgehäusen verbliebenen Eier und kleinen Larve, können zu einer Bestückung dienen, sodass neue Eier oder Zuchttiere nicht unbedingt zugesetzt werden müssen.

Es ist allerdings auch möglich, dass die Druckschutzgehäuse für mehrere nachfolgende Zuchtvorgänge im Zuchtbehälter verbleiben. In diesem Fall wird das in den Druckschutzgehäusen befindliche Füllmaterial mittels beispielsweise eines Luftstrahls oder Wasser durch die Öffnungen im Druckschutzgehäuse ausgespült und durch die Ausgabeöffnung der Zuchtbehälters ausgegeben.

Zur Auflockerung und/oder besseren Durchlüftung des Füllmaterials können auch Durchmischungsvorrichtungen in dem Zuchtbehälter vorgesehen sein. Eine Durchmischungsvorrichtung kann beispielsweise ein oder mehrere Rührwerke umfassen. Eine Durchmischungsvorrichtung kann anstelle von Druckschutzelementen, oder auch in Kombination mit Druckschutzelementen in dem Behälter vorgelegt werden.

Um die Belüftung des Füllmaterials zu verbessern kann der Zuchtbehälter mit geeignete Belüftungsvorrichtungen, beispielsweise Luftdüsen, laterale Öffnungen, oder Belüftungsschläuche versehen sein.

Luft oder Sauerstoff kann durch diese Belüftungsvorrichtungen in den Zuchtbehälter geblasen werden, oder passiv eindringen. Die eingeleitete Luft kann überschüssige Wärme und Kohlendioxid aus dem Füllmaterial abführen.

Die Luftzufuhr erfolgt vorzugsweise dem unteren Bereich des Zuchtbehälters. Es ist allerdings auch möglich, dass Luft auf verschiedenen Ebenen, beziehungsweise Höhen, des Zuchtbehälters zugeführt wird.

Die zugeführte Luft steigt auf und führt die von den Larven produzierte Wärme und das Kohlendioxid nach oben ab.

Eine Luftzufuhr, die unterhalb der Druckschutzelemente erfolgt, hat den Vorteil, dass sich frische Luft in den durch die Druckelemente geschaffenen Schutzzonen ansammeln kann. Dies ist besonders vorteilhaft, wenn die Druckschutzelemente dachförmige Umleitstrukturen sind. In dieser Ausführung kann sich die zugeführte Luft unterhalb der dachförmigen Strukturen ansammeln und Lufttaschen bilden.

Durch eine angemessene Regulierung der Luftzufuhr und/oder der Temperatur der zugeführten Luft kann die Füllmasse je nach Bedarf geheizt oder gekühlt werden.

Die Ausgabeöffnung des Zuchtbehälters kann eine Öffnung an einer unteren Wand, oder im Boden des Zuchtbehälters sein.

Vorzugsweise ist der untere Bereich des Zuchtbehälters trichterförmig ausgebildet, wobei die untere Öffnung des trichterförmigen Bereichs der Ausgabeöffnung entspricht.

Es ist allerdings auch möglich, dass die im unteren Bereich des Zuchtbehälters befindliche Ausgabeöffnung an einem Gehäuse einer sich im Innern des Zuchtbehälters befindlichen Fördervorrichtung angeordnet ist.

In diesem Ausführungsbeispiel ist die im Innern des Zuchtbehälters befindliche Fördervorrichtung dazu ausgerichtet, Füllmaterial und/oder Druckschutzgehäuse aus dem unteren Bereich des Zuchtbehälters in Richtung der Eingabeöffnung zu transportieren. Das Gehäuse umgibt die Fördervorrichtung und grenzt diese vom Füllmaterial ab, wobei das Füllmaterial im Zuchtbehälter lediglich durch die Ausgabeöffnung ins Innere des Gehäuses eindringen kann.

Der Zuchtbehälter kann Bestandteil eines Zuchtsystems, das zusätzlich eine Trennvorrichtung zur Auftrennung des geernteten Materials aufweist, sein. Die Trennvorrichtung kann beispielsweise ein Sieb, oder einer Serie von Sieben mit unterschiedlichen Maschenweiten, die Füllmaterial entsprechend der Grösse trennen, sein. So können herangewachsene, zur Tierfütterung geeignete Insektenlarven von kleinen Larven und Eiern, sowie von verbleibendem Substrat und Abfallmaterial separiert werden.

Das Zuchtsystem kann weiters eine Fördervorrichtung aufweisen, die geerntetes Füllmaterial von der Ausgabeöffnung zur Trennvorrichtung transportiert.

Die Erfindung betrifft des Weiteren ein Verfahren zur Anzucht von Insektenlarven, die folgende Schritte umfassend:
a. Befüllen oder Aufstocken des Zuchtbehälters mit Füllmaterial, welches Substrat für die Zuchttiere, sowie Insekteneier und/oder Zuchttiere umfasst, durch die obere Eingabeöffnung,
b. Inkubation des Füllmaterials im Zuchtbehälter,
c. Ermöglichen eines kontrollierten Durchflusses des Füllmaterials durch den Zuchtbehälter mittels des Regulierens der Füllmaterialausgabe durch die Ausgabeöffnung,
d. Ernten des durch die Ausgabeöffnung ausgegebenen Füllmaterials.

Die Schritte b und c können zeitgleich durchgeführt werden. Es ist allerdings auch möglich, dass die Inkubation während einer definierten Periode erfolgt und ein Durchfluss während kurzer Intervalle erfolgt. Da der Durchfluss durch die Ausgabe des Füllmaterials ermöglicht wird, erfolgen Ernte und Durchfluss gleichzeitig. In einem kontinuierlichen Durchflussverfahren erfolgen somit Schritte b, c und d gleichzeitig.

Zur Bestückung des Zuchtbehälters mit Insektenzuchttieren kann das Füllmaterial entweder mit Insekteneiern, kleinen Larven oder adulten Insekten versetzt werden. Zur Aufstockung des Zuchtbehälters ist es allerdings auch möglich Substrat ohne Zuchttiere einzufüllen, da die Zuchttiere die bereits in dem Zuchtbehälter vorliegenden Füllmaterial vorgelegten Tiere das Auffüllmaterial besiedeln können.

### Kurze Beschreibung der Figuren

Die Erfindung wird anhand der beigefügten Figuren näher erläutert, wobei zeigen
Fig. 1A eine dreidimensionale Ansicht einer möglichen Ausführungsform des Zuchtsystems für Insektenlarven mit giebelförmigen Druckschutzelementen;
Fig. 1B eine Querschnittansicht der in Figure 1A dargestellten Ausführungsform des Zuchtsystems;
Fig. 1C eine Querschnittansicht einer Ausführungsform eines Zuchtbehälters mit einer zentralen Fördervorrichtung;
Fig. 2A eine dreidimensionale Teilansicht eines Ausführungsbeispiels eines geöffneten Schutzbehälters, aus dem vier Tragringe giebeldachförmiger Druckschutzelemente herausgenommen sind;
Fig. 2B eine dreidimensionale Ansicht eines Tragringes mit giebeldachförmigen Druckschutzelementen;
Fig. 3A eine dreidimensionale Draufsicht auf einen Querschnitt eines Ausführungsbeispiels des Zuchtbehälters, in dem die Regulierungsvorrichtung der Ausgabe des Füllmaterials in geöffneter Stellung dargestellt ist;
Fig. 3B eine dreidimensionale Draufsicht auf einen Querschnitt eines Ausführungsbeispiels des Zuchtbehälters, in dem die Regulierungsvorrichtung der Ausgabe des Füllmaterials in halb-offener Stellung dargestellt ist;
Fig. 4 eine Querschnittansicht einer möglichen Ausführungsform des Zuchtsystems für Insektenlarven, in dem Druckgehäuse zur Anzucht der Larven vorgesehen sind;
Fig. 5A bis 5E verschiedene mögliche Ausführungsformen perforierter Druckschutzgehäuse;

### Wege zur Ausführung der Erfindung

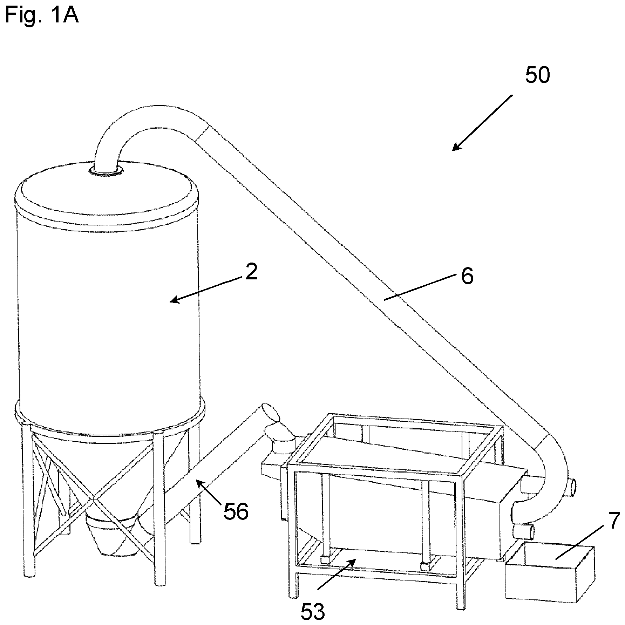

Erfindungsgemässe Ausführungsbeispiele des beanspruchten Zuchtbehälters und des Zuchtsystems sind schematisch in Figuren 1A bis 5E dargestellt.

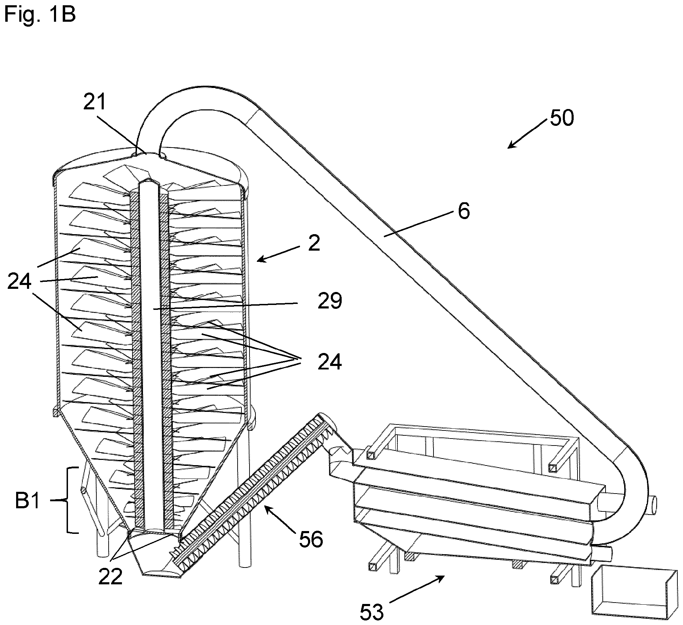

In Figur 1A ist ein Zuchtsystem 50 zur Anzucht von Insektenlarven mit einem silo-förmigen Zuchtbehälter 2, einer externen Förderanlage 56 und einer Trennvorrichtung 53 dargestellt. Der Zuchtsilo weist einen trichterförmigen unteren Bereich B1 auf, der in die Ausgabeöffnung 22 mündet (Figur 1B).

Das durch die Ausgabeöffnung 22 ausgegebene Füllmaterial wird mittels der externen Förderanlage 56 zur Trennvorrichtung 53 befördert. Dies kann, wie in Figur 1B dargestellt, beispielsweise mittels einer Förderschnecke erfolgen.

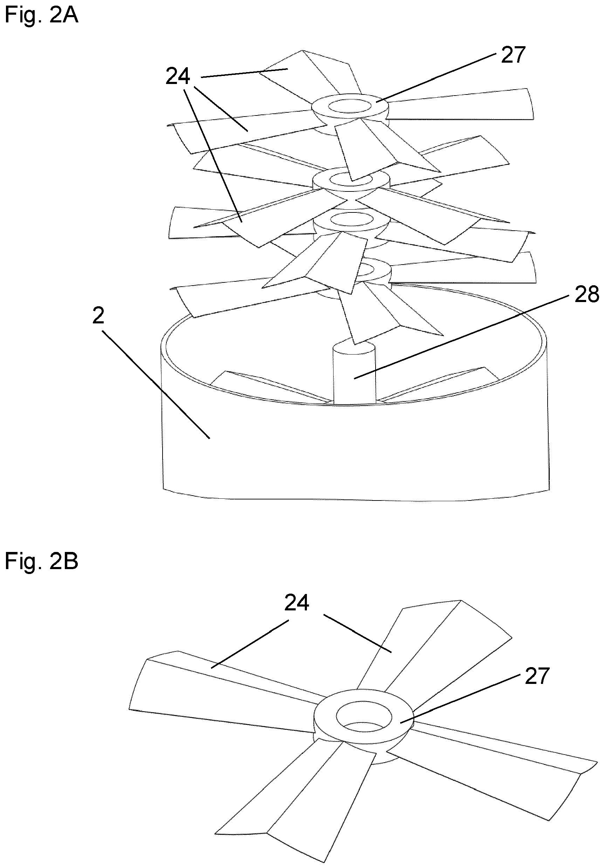

Der in Figur 1B dargestellte Zuchtbehälter weist in seinem Zentrum eine zentrale Säule 29 auf, auf der Tragringe 27, wie in Figur 2A veranschaulicht, aufgesteckt sind.

Wie in Figur 2B gezeigt, sind an jedem Tragring 27 in diesem Ausführungsbeispiel vier giebeldachförmige Druckschutzelemente 24 angebracht.

Es ist allerdings auch möglich, eine andere Anzahl an Druckschutzelementen 24 an die Tragringe anzubringen.

Die Form der angebrachten Druckschutzelemente kann ebenfalls variieren. Es ist weiters möglich Druckschutzelemente unterschiedlicher Formen and einem Tragring anzubringen.

In einem möglichen Ausführungsbeispiel rotiert die zentrale Säule 29 und treibt eine Rotation der Druckschutzelemente 24 an. Die Rotation der Druckschutzelemente begünstigt die Auflockerung des Füllmaterials, sowie die Vermischung des Substrats.

Unterhalb der Druckschutzelemente 24 bilden sich Schutzzonen, in denen der durch das Gewicht der darüberliegenden Füllmasse bedingte Druck reduziert ist.

Die giebelförmige Ausführung der Druckelemente 24 bietet den zusätzlichen Vorteil, dass sich unterhalb der Giebel Lufttaschen bilden können. Heranwachsende Larven mancher Insektenarten, beispielsweise Mehlwürmer, gedeihen an der Schnittstelle zwischen Substrat und Luft besonders gut und sammeln sich vorwiegend in diesen Bereichen unterhalb der Giebel an.

Der Zuchtbehälter weist eine Eingabeöffnung 21 im oberen Teil des Behälters auf. In dem dargestellten Beispiel ist die Eingabeöffnung 21 an eine Röhre angeschlossen, durch die, wie nachstehend beschrieben Insekten zur Bestückung zugeführt werden können. Zusätzlich kann durch die Eingabeöffnung 21 frisches Substrat und, optional, Feuchtfutter eingefüllt werden. Zu diesen Zweck kann die Röhre entfernt werden.

Die Ausgabeöffnung 22 des in Figuren 1A und 1B dargestellten Ausführungsbeispiels wird von der sich verjüngenden Struktur des trichterförmigen unteren Bereichs B1 gebildet.

Die Ausgabeöffnung dieses Ausführungsbeispiels ist vorzugsweise mit einer Regulierungsvorrichtung 28 ausgestattet. Die Regulierungsvorrichtung kann eine konventionelle Siloverschlussklappe, beispielsweise eine Drehverschlussklappe sein. Vorzugsweise ist die Regulierungsvorrichtung derart verstellbar, dass die Geschwindigkeit des Durchflusses des Füllmaterial kontrolliert werden kann.

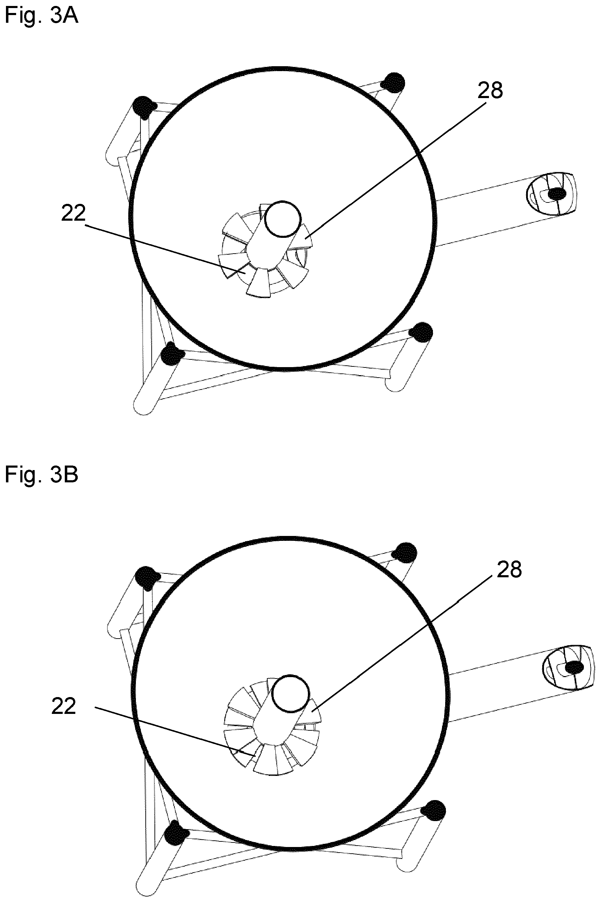

Zu diesem Zweck eignet sich beispielsweise die in Figuren 3A und 3B abgebildete Regulierklappe, die aus zwei überlagerten Klappelementen besteht. Die Klappelemente der Regulierklappe können zueinander unterschiedliche Winkelpositionen einnehmen. Die beiden Klappelemente sind in Bezug aufeinander rotativ beweglich.

Wie in Figur 3A abgebildet können die beiden Klappelemente derart überlagert sein, dass die Ausgabeöffnung 22 zumindest teilweise frei gegeben wird.

In Figur 3B nehmen die Klappelemente eine Stellung ein, in der die Ausgabeöffnung 22 von Flächen beider Klappelemente abgedeckt ist. Dies entspricht einer halb-offenen Stellung, in der der Materialdurchfluss verringert ist.

In einer geschlossenen Stellung der in Figuren 3A und 3B dargestellten Regulierungsvorrichtung nehmen die beiden Klappelemente in Bezug aufeinander eine Winkelposition ein, in der die Ausgabeöffnung vollständig abgedeckt ist (nicht dargestellt).

Die in Figur 1B dargestellte Trennvorrichtung ist eine Anlage mit seriellen Sieben, in derer Materialien und Tiere entsprechend ihrer Grösse in verschiedene Lagen getrennt werden. Die Trennvorrichtung weist drei Lagen auf.

In der obersten Lage werden Larven über einer vorbestimmte Erntegrösse abgetrennt. Die Maschengrösse des obersten Siebes lässt Tiere und Materialien geringerer Grösse durchfallen.

In der mittleren Lage werden kleine Larven aufgefangen. Diese Larven können zur Wiederbestückung dem Zuchtbehälters zugeführt werden. Wie in Figuren 1A und 1B dargestellt, können die in der mittleren Lage abgetrennten Larven mittels einer Zuführvorrichtung 6, beispielsweise eine Förderwelle in einem Gehäuse, dem Zuchtbehälter 2 zur Bestückung zugeführt werden.

Die unterste Lage der in Figur 1B dargestellten Trennvorrichtung fängt Abfallmaterialien, beispielsweise Kot und Substratreste auf, die verworfen werden können.

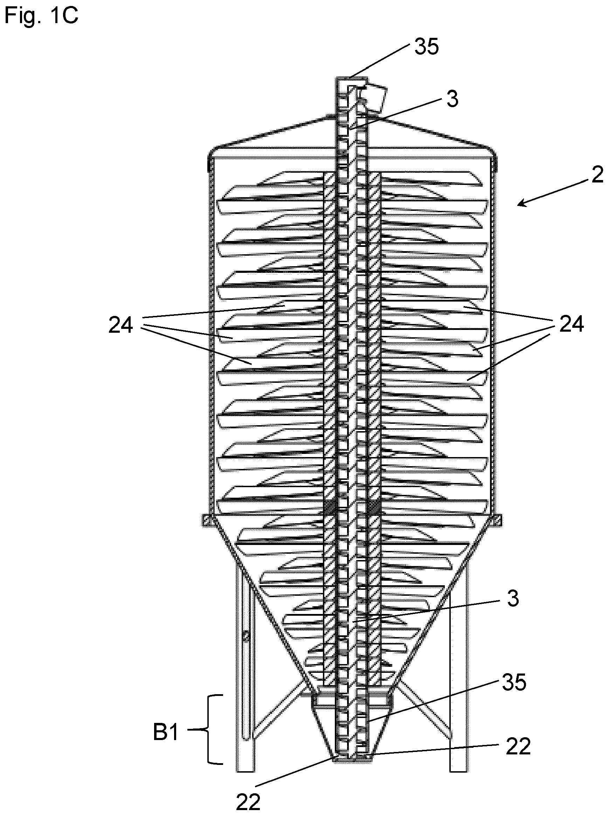

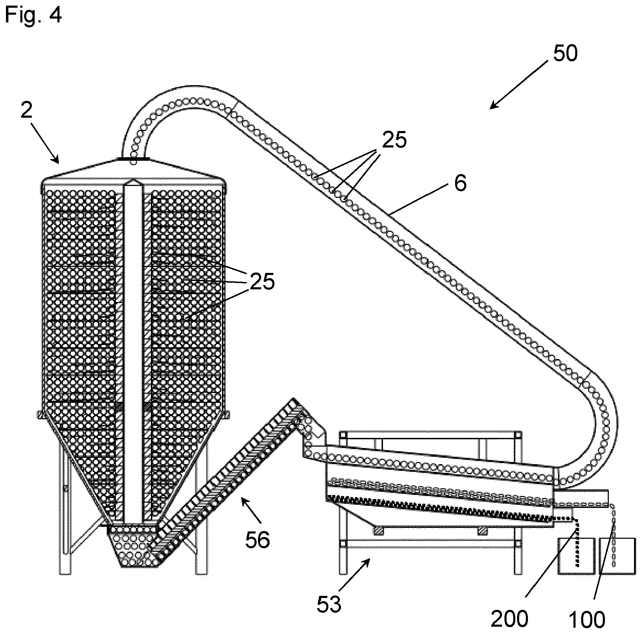

In Figur 1C ist ein Ausführungsbeispiel mit einer zentral angeordneten Fördervorrichtung abgebildet. Die Fördervorrichtung ist im dargestellten Beispiel eine Förderwelle 3, die in einem Gehäuse 35 angeordnet ist. In Figur 1C ist die zentrale Fördervorrichtung in einen Zuchtbehälter mit giebelförmigen Druckschutzelementen dargestellt. Eine zentrale Fördervorrichtung kann allerdings auch in andere Arten von beanspruchten Zuchtbehältern eingebaut werden. Beispielswiese ein mit Druckschutzgehäusen befüllbaren Zuchtbehälter, wie zum Beispiel in Figur 4 vorgestellt, kann eine zentrale Fördervorrichtung aufweisen.

Wenn der Zuchtbehälter eine zentrale Förderaufrichtung aufweist, sollte die im unteren Bereich B1 des Behälters angeordnete Ausgabeöffnung 22 von der Gehäuseöffnung der Fördervorrichtung vorgegeben sein. Aus diesem Grund ist das Gehäuse 35 der Fördervorrichtung in Bezug auf den Boden des Zuchtbehälters angehoben, um einen Zugriff auf das Füllmaterials, und/oder ein Eindringen des Füllmaterials durch die Ausgabeöffnung 22 in die Fördervorrichtung zu ermöglichen.

Die zentrale Fördervorrichtung ist vorzugsweise derart ausgerichtet, dass durch die Ausgabeöffnung erhaltenes Material nach oben, das heisst in Richtung der Eingabeöffnung 21 des Zuchtbehälters 2, zu transportieren.

Der Zuchtbehälter eines Ausführungsbeispiels mit zentraler Fördervorrichtung mündet demnach nicht in eine Ausgabeöffnung 22, sondern weist einen Boden auf. Die Ausgabeöffnung 22 ist im Inneren des Behälters angeordnet.

Das in Figuren 1A, 1B und 1C dargestellte Zuchtsystem ist vor allem für Insektenarten, die auf eine gute Substratdurchlüftung angewiesen sind, wie zum Beispiel Mehlwürmer, geeignet.

Figur 4 zeigt ein alternatives Ausführungsbeispiel eines Zuchtsystems 50, das besonders für Insektenlarven, die gut in feuchtem Substrat gedeihen, geeignet ist. Dieses Zuchtsystem eignet sich beispielsweise zur Anzucht der Larven der schwarzen Soldatenfliege. Lufttaschen, wie sie unter den giebelförmigen Druckschutzelementen gebildet werden können, sind für diese Larven nicht erforderlich.

Der Zuchtbehälter 2 dieses Ausführungsbeispiels ist mit Druckschutzgehäusen 25 versehen, die eine Vielzahl von Öffnungen aufweisen. Die abgebildeten Druckschutzgehäuse 25 sind hohle Kugeln mit Öffnungen. Die Form der Druckschutzgehäuse 25, und/oder der Form der Öffnungen ist allerdings nicht besonders limitiert.

Druckschutzgehäuse 25 sind hohle Strukturen mit Öffnungen, durch die Füllmaterial eindringen und auslaufen kann. Druckschutzgehäuse 25 sind dynamische Strukturen, die in den Zuchtbehälter eingefüllt und von diesem ausgegeben werden können.

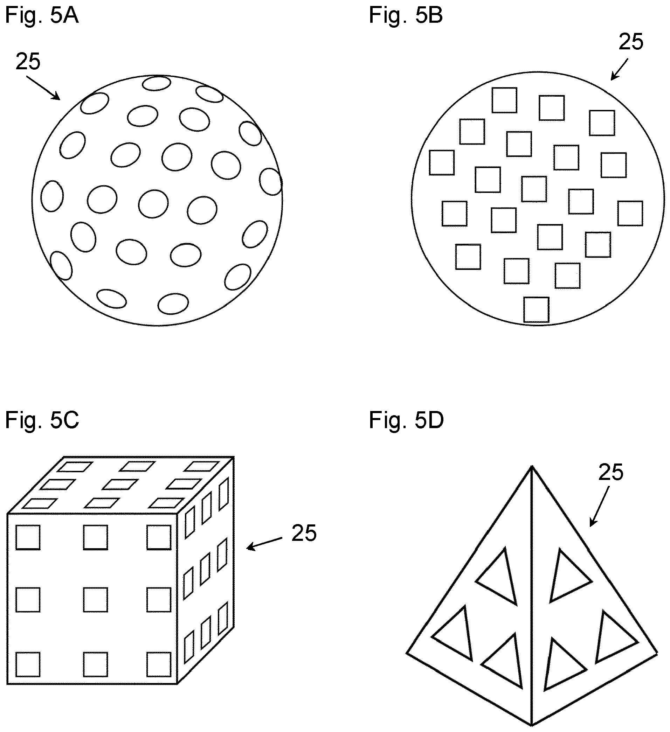

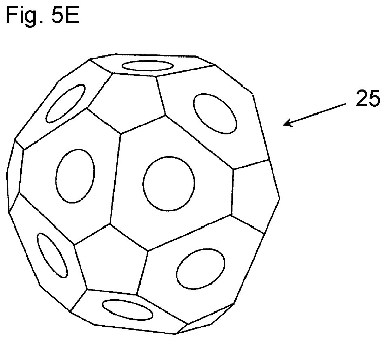

Wie in Figur 5A und B dargestellt, können Druckschutzgehäuse 25 perforierte, hohle Bälle oder Kugeln sein. Druckschutzgehäuse 25 können auch würfelförmig sein, oder eine pyramidale Struktur aufweisen, wie dies in Figuren 5B und 5C dargestellt ist. Druckschutzgehäuse 25 können beliebige hohle Polyeder mit mehreren Öffnungen sein, Figur 5E.

Druckgehäuse 25 können mit nassem Substrat und, optional mit Insekteneiern, kleinen Larven, oder adulten Insekten, befüllt werden und in den Zuchtbehälter eingegeben werden. Zusätzlich kann der Zuchtbehälter mit vorzugsweise nassem Substrat befüllt werden. Das zusätzliche Substrat dient dazu die Zwischenräume zwischen den Druckschutzgehäusen 25 auszufüllen.

Für manche Insektenarten, wie zum Beispiel für die Larven der schwarzen Soldatenfliege, ist es von Vorteil, wenn ein Zuchtbehälter mit befüllten Druckgehäusen angefüllt wird und bis zum Zeitpunkt der Ernte statisch, das heisst ohne einen Durchfluss des Füllmaterials durch den Zuchtbehälter, inkubiert wird. Die Larven des gesamten Behälters werden dann in einem Erntegang ausgegeben.

Die Ernte der Larven kann durch eine Ausgabe der Druckschutzgehäuse erfolgen. Das Material und die Larven im Inneren der Druckschutzgehäuse 25 kann dann durch einen Wasserstrahl oder einen Luftstrahl aus den Druckschutzgehäusen 25 herausgespült werden, sodass die Larven geerntet werden können.

Das Material und die Larven können von den ausgegebenen Druckschutzgehäusen 25 allerdings auch mittels der in Figur 4 abgebildeten Trennvorrichtung 53 getrennt werden. Die Trennvorrichtung 53 umfasst eine Serie von Sieben mit unterschiedlicher Maschenweite, die verschiedene Lagen der Vorrichtung trennen. Das oberste Sieb in Figur 4, das den Boden der obersten Lage bildet, ist vorzugsweise ein Schüttelsieb. Dieses Sieb weist die grösste Maschenweite auf.

Ein Schütteln der Druckschutzgehäuse 25 bewirkt ein Auslaufen des in den Druckschutzgehäusen befindlichen Materials mit den darin enthaltenen Larven durch die Öffnungen der Druckschutzgehäuse. Die Maschengrösse sollte gewählt sein, um die Druckschutzgehäuse 25 zurückzuhalten, aber das Material und die Zuchttiere in die nächste, mittlere Lage fallen zu lassen.

Das Sieb der mittleren Lage weist vorzugsweise eine Maschengrösse auf, die Larven einer vorbestimmten gewünschten Grösse zurückhält, aber kleineres Material und kleinere Larven passieren lässt.

Die erntebereiten Larven können somit aus der mittleren Lage abgeerntet werden. Kleineres Material kann verworfen werden. Die in der obersten Lage abgetrennten Druckschutzgehäuse 25 können dem Zuchtbehälter 2 wieder zugeführt werden.

Die Druckschutzgehäuse 25 können vorher wieder mit Substrat und Zuchttieren, beziehungsweise Eiern bestückt werden.

Andererseits ist es auch möglich, die leeren Druckschutzgehäuse 25 in den Behälter einzufüllen und separat mit Eiern oder Zuchttieren bestücktes Substrat zuzuführen. Das bestückte Substrat kann dann in dem Zuchtbehälter 2 durch die Öffnungen der Druckschutzgehäuse in die Druckschutzgehäuse 25 einlaufen. Es ist in diesem Fall vorteilhaft, eine Durchmischungsvorrichtung vorzusehen, die dazu ausgestattet ist, die dynamischen Druckschutzgehäuse 25 in dem Zuchtbehälter mit dem Substrat zu vermischen. Die Durchmischungsvorrichtung kann beispielsweise ein Rührwerk sein.

Es ist allerdings auch möglich, dass die Druckschutzgehäuse 25 bei der Ernte nicht durch die Ausgabeöffnung 22 ausgegeben werden. Zur Ernte kann der Zuchtbehälter samt den Druckschutzgehäusen 25 beispielsweise mit Wasser oder einem starken Luftstrahl durchflutet werden, sodass das in den Druckschutzgehäusen 25 befindliche Material und die Larven ausgespült und durch die Ausgabeöffnung 22 ausgegeben werden.

Die in den Figuren offenbarten Ausführungsbeispiele des Zuchtbehälters und des Zuchtsystems sind bevorzugte Ausführungsbeispiele der beanspruchten Erfindung. Es ist allerdings klar, dass verschiedene Abänderungen und/oder Modifikationen dieser bevorzugten Ausführungsform möglich und für die Fachperson offensichtlich sind. Diese Änderungen schliessen auch verschiedene Kombinationen der vorgestellten Vorrichtungen mit verschiedenen Ausführungen des Zuchtbehälters ein. Solche Abänderungen und/oder Modifikationen liegen im Sinne dieser Erfindung und können vorgenommen werden, ohne vom Anwendungsbereich der vorliegenden Erfindung abzuweichen. Es ist daher beabsichtigt, derartige Abänderungen und/oder Modifikationen ebenfalls durch die nachfolgenden Ansprüche abzudecken.

## Patentansprüche

1. Eine Zuchtvorrichtung (1) zur Anzucht von Insektenlarven aufweisend einen Zuchtbehälter (2) mit einer oberen Eingabeöffnung (21) zum Befüllen des Zuchtbehälters mit Füllmaterial und mit in einer im unteren Bereich (B1) des Zuchtbehälters befindlichen Ausgabeöffnung (22), wobei die Eingabeöffnung (21) und die Ausgabeöffnung (22) derart angeordnet sind, dass ein Füllmaterialfluss von der Eingabeöffnung zur Ausgabeöffnung erstellt wird, und wobei der Zuchtbehälter eine Vielzahl von formstabilen und/oder formresistenten Druckschutzelementen (24, 25) aufweist, die dazu ausgebildet sind, den Druck, der von einem Teil der Masse des Füllmaterials auf einen unterhalb dieses Teils liegenden Teil der Masse des Füllmaterials ausgeübt wird, zu reduzieren.

2. Die Zuchtvorrichtung nach Anspruch 1, wobei die Ausgabeöffnung mit einer Regulierungsvorrichtung (28), beispielsweise einer verstellbaren Klappe, ausgestattet ist, mittels derer die Füllmaterialausgabe reguliert und/oder gestoppt werden kann.

3. Die Zuchtvorrichtung nach einem der Ansprüche 1 oder 2, weiters aufweisend eine Belüftungsvorrichtung, beispielsweise Luftdüsen, laterale Öffnungen, oder Belüftungsschläuche, die eine Belüftung von Füllmaterial im Zuchtbehälter ermöglichen.

4. Die Zuchtvorrichtung nach einem der Ansprüche 1 bis 3, wobei die Druckschutzelemente (24) im Zuchtbehälter (2) angebracht sind, beispielsweise mittels einer im Innenraum des Zuchtbehälters angeordnete Trägerstruktur.

5. Die Zuchtvorrichtung nach einem der Ansprüche 1 bis 4, wobei die im Zuchtbehälter angebrachten Druckschutzelemente dachförmige Umleitstrukturen sind, beispielsweise giebelförmige, kegeldachförmige oder gewölbte Elemente, deren Form einen Abfluss des über dem Druckschutzelement befindlichen Füllmaterials entlang der Oberfläche des Druckschutzelements bewirkt.

6. Die Zuchtvorrichtung nach einem der Ansprüche 1 bis 3, wobei die Druckschutzelemente mit Füllmaterial befüllbare Druckschutzgehäuse sind, die in den Zuchtbehälter eingefüllt und die durch die Ausgabeöffnung des Zuchtbehälters entlassen werden können.

7. Die Zuchtvorrichtung nach Anspruch 6, wobei die Druckschutzgehäuse eine Vielzahl von Öffnungen aufweisen, die ein Eindringen und Auslaufen von Füllmaterial ermöglichen.

8. Die Zuchtvorrichtung nach einem der Ansprüche 1 bis 7, wobei der untere Bereich B1 des Zuchtbehälters, der vorzugsweise trichterförmig ausgebildet ist, in die Ausgabeöffnung mündet.

9. Die Zuchtvorrichtung nach einem der Ansprüche 1 bis 7, wobei der Zuchtbehälter weiters eine im Innenraum des Zuchtbehälters befindliche Fördervorrichtung (3) mit einem Gehäuse (35) umfasst, wobei die Fördervorrichtung dazu ausgerichtet ist, Füllmaterial und/oder Druckschutzgehäuse aus dem unteren Bereich B1 des Zuchtbehälters in Richtung der Eingabeöffnung (21) zu transportieren, und wobei die Ausgabeöffnung (22) des Zuchtbehälters derart am Gehäuse der Fördervorrichtung angeordnet ist, dass das Füllmaterial und/oder die Druckschutzgehäuse (25) aus dem unteren Bereich B1 in das Innere des Gehäuses eindringen können.

10. Ein System (50) zur Anzucht von Insektenlarven umfassend einen Zuchtbehälter (2) nach einem der Ansprüche 1 bis 9, weiters aufweisend eine Trennvorrichtung (53) zum Separieren von Insektenlarven und Abfallmaterialien basierend auf deren Grösse.

11. Das System nach Anspruch 10, weiters umfassend eine ausserhalb des Zuchtbehälters angeordnete Fördervorrichtung (56) zur Beförderung des aus dem Zuchtbehälter ausgegebenen Materials zur Trennvorrichtung (53).

12. Ein Verfahren zur Anzucht von Insektenlarven in einer Zuchtvorrichtung nach einem der Ansprüche 1 bis 9, oder in einem System nach einem der Ansprüche 10 oder 11, die folgenden Schritte umfassend:
a. Befüllen oder Aufstocken des Zuchtbehälters mit Füllmaterial, welches Substrat für die Zuchttiere, sowie Insekteneier und/oder Zuchttiere umfasst, durch die obere Eingabeöffnung (21),
b. Inkubieren des Füllmaterials im Zuchtbehälter,
c. Ermöglichen eines kontrollierten Durchflusses des Füllmaterials durch den Zuchtbehälter mittels des Regulierens der Füllmaterialausgabe durch die Ausgabeöffnung (22),
d. Ernten des durch die Ausgabeöffnung (22) ausgegebenen Füllmaterials.

13. Das Verfahren nach Anspruch 12, wobei Schritt b der Inkubation und Schritt c des kontrollierten Durchflusses gleichzeitig durchgeführt werden.

14. Das Verfahren nach Ansprüchen 12 oder 13, wobei der Durchfluss ausschliesslich durch die auf das Füllmaterial einwirkende Gravitationskraft verursacht wird.

15. Das Verfahren nach Ansprüchen 12 bis 14, wobei das mit Insekteneiern und/oder Zuchttieren bestückte Substratmaterial in Druckschutzgehäusen vorgelegt wird und gemeinsam mit den Druckschutzgehäusen in den Zuchtbehälter gefüllt wird.

16. Das Verfahren nach Ansprüchen 12 bis 15, weiters umfassend einen zusätzlichen, dem Schritt c nachfolgenden Schritt d des Separierens, beispielsweise durch Sieben, der im Füllmaterial herangewachsenen Larven einer vorbestimmten Grösse von dem restlichen geernteten Füllmaterial.
